# EUROPEAN PATENT SPECIFICATION

(11) **EP 1 529 502 B1**
(45) Date of publication and mention of the grant of the patent: **06.03.2013**
(21) Application number: 04256037.5
(22) Date of filing: 30.09.2004
(51) Int. Cl.: A61F 5/00

(54) **Implantable band with transverse attachment mechanism**
Implantierbares Band mit einer transversalen Verbindungsvorrichtung
Bande implantable avec mécanisme de fixation transversal

(30) Priority: 30.09.2003 US 677088
(43) Date of publication of application: 11.05.2005
(73) Proprietor: ETHICON ENDO-SURGERY, INC., Cincinnati, Ohio 45242 (US)
(72) Inventor: Byrum, Randal T., Milford Ohio 45150 (US); Jambor, Kristin L., Cincinnati Ohio 45208 (US); Crawford, Norman, Washington CH Ohio 43160 (US)
(74) Representative: Tunstall, Christopher Stephen

(56) References cited:
- EP-A- 1 396 242
- WO-A-03/101352
- US-A- 5 449 368
- US-A1- 2004 267 292

## Description

### Technical Field

This present invention relates generally to a surgically implantable band for encircling an anatomical passageway, and is particularly directed to an adjustable gastric band for encircling the stomach for the control of obesity. The invention will be specifically disclosed in connection with an improved attachment mechanism for an adjustable gastric band.

### Background Of The Invention

Since the early 1980s, adjustable gastric bands have provided an effective alternative to gastric bypass and other irreversible surgical weight loss treatments for the morbidly obese. The gastric band is wrapped around an upper portion of the patient's stomach, forming a stoma that is less than the normal interior diameter of the stomach that restricts food passing from an upper portion to a lower digestive portion of the stomach. When the stoma is of the appropriate size, food held in the upper portion of the stomach provides a feeling of fullness that discourages overeating.

In addition to a latched position to set the diameter of the gastric band, adjustablity of gastric bands is generally achieved with an inwardly directed inflatable balloon, similar to a blood pressure cuff, into which fluid, such as saline, is injected through a fluid injection port to achieve a desired diameter. The balloon is typically deflated or only partially inflated when first placed in the body to allow for body adjustments and healing around the new band site. Since adjustable gastric bands may remain in the patient for long periods of time, the fluid injection port is typically installed subcutaneously to avoid infection, for instance in front of the sternum. Following the initial implantation, the surgeon may adjust the band by loosing or tightening depending on the patients' needs. Adjusting the amount of fluid in the adjustable gastric band is achieved by inserting a Huber tip needle through the skin into a silicone septum of the injection port. Once the needle is removed, the septum seals against the hole by virtue of compressive load generated by the septum. A flexible conduit communicates between the injection port and the adjustable gastric band.

An attachment mechanism for the adjustable gastric band has to provide an initial sizing of the stoma of the stomach. One generally known attachment is to suture ends of the adjustable gastric band. Another generally known attachment includes one end of the gastric band terminating in a flexible conduit that has a flared portion that is drawn through an opening in a second end of the gastric band and then sutured to the encircling band portion - securing the band to the stomach. After the sutures are in place, the injection port is anchored at a convenient location.

While these known approaches are effective in securing the gastric band, further improvements are desired that simplify the clinical implantation procedure, that provide long-term reliability, and that facilitate readjustment or removal.

While sutures have been relied on as the most positive connection in the past, it is desirable to have a secure attachment that does not require sutures, yet does not require a large force to create the secure attachment. Otherwise, it may be difficult to adequately grip and perform the attachment with laparoscopic instruments. Consequently, a significant need exists for an adjustable gastric band having an improved attachment mechanism.

US 5 449 368 fails to teach a transverse attachment mechanism as defined in claim 1.

### Summary of The Invention

The present invention addresses these and other problems in the prior art, by providing an adjustable gastric band device as defined in claim 1 that is engaged with less force, thereby facilitating implementation with laparoscopic instruments, yet the attachment remains secure over long term use.

A general object of this invention is to provide an adjustable gastric band having a transverse attachment mechanism.

Another object of this invention is to provide a readily reversible adjustable gastric band which can be fastened and unfastened without reducing the holding strength of the attachment mechanism.

It is another object of the present invention to provide an adjustable gastric band in which the force necessary to disengage the ends of the adjustable gastric band is nominal, and is not in same direction as the longitudinal holding forces so as not to separate the two ends.

To achieve the foregoing and other objects, and in accordance with the purposes of the present invention as described herein, there are described adjustable gastric bands with transverse attachment mechanisms connecting the two ends together. The transverse attachment mechanisms include a dovetail connection, and a hook with a circuitous path.

Further novel features and other objects of the present invention will become apparent from the following detailed description, discussion and the appended claims, taken in conjunction with the drawings.

### Brief Description of the Figures

The accompanying drawings, which are incorporated in and constitute a part of this specification, illustrate embodiments of the invention, and, together with the general description of the invention given above, and the detailed description of the embodiments given below, serve to explain the principles of the present invention.
FIG. 1 is a diagrammatic drawing showing an adjustable gastric band wrapped around an upper part of a stomach.
FIG. 2 is a cross sectional view of the adjustable gastric band of FIG 1 taken along line 2-2.
FIG. 3 is a perspective view of an adjustable gastric band having a dovetail transverse attachment mechanism.
FIG. 4 is a top view of the adjustable gastric band shown in FIG. 3.
FIG. 5 is a perspective view of an adjustable gastric band having a projected dovetail transverse attachment mechanism shown unattached.
FIG. 6 is a side view of the adjustable gastric band of FIG. 5 with the two ends of the projected dovetail transverse attachment mechanism attached together.
FIG. 7 is top view of the adjustable gastric band shown in FIG. 6.
FIG. 8 is a perspective view of an adjustable gastric band with a hook transverse attachment mechanism shown unattached.
FIG. 9 is a perspective of the adjustable gastric band of FIG. 8 with the hook transverse attachment mechanism attached together.
FIGS. 10A-D are a sequence of side views in cross section taken along plane 10-10 of FIG. 9 illustrating engagement of the hook end to the buckle.
FIG. 11 depicts an alternate embodiment of the hook member of FIG. 8.

Reference will now be made in detail to the present preferred embodiment of the invention, an example of which is illustrated in the accompanying drawings.

### Detailed Description of Embodiments of the Invention

In the following description, like reference characters designate like or corresponding parts throughout the several views. Also, in the following description, it is to be understood that terms such as front, back, inside, outside, and the like are words of convenience and are not to be construed as limiting terms. Terminology used in this patent is not meant to be limiting insofar as devices described herein, or portions thereof, may be attached or utilized in other orientations. Referring in more detail to the drawings, the invention will now be described.

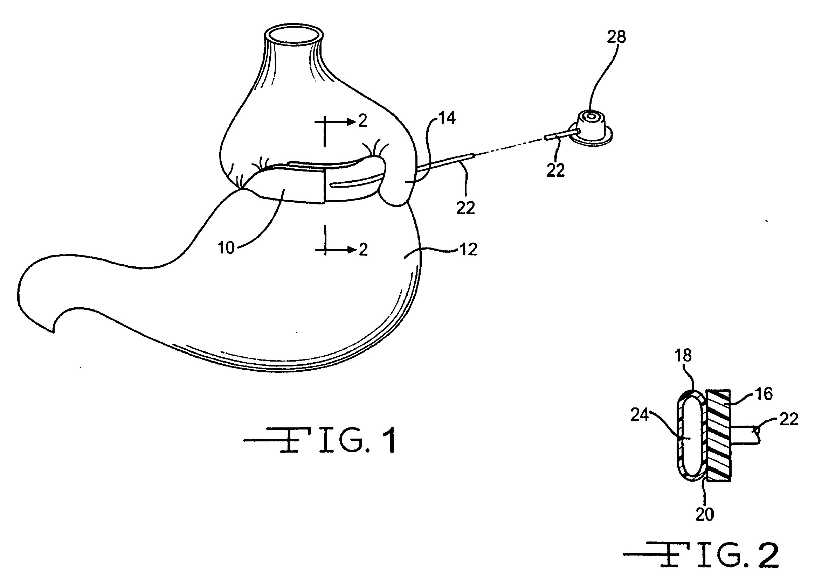

Referring to Fig. 1, an adjustable gastric band 10 is shown wrapped around an upper portion of a stomach 12, kept in place by attaching the two ends together and extending a portion 14 of the stomach 12 over the adjustable gastric band 10 by suturing portion 14 to the stomach. Referring also to Fig. 2, the adjustable gastric band 10 includes a non-extensible strap 16 and an inflatable balloon 18, made of a medical grade silicone polymer or any other suitable material, is carried by the inner surface 20 of the strap 16. The balloon 18 may be secured to the inner surface 20 in any well known manner, or even made of unitary construction with the strap 16, although the strap 16 may typically be formed of a different material.

One end of a flexible conduit 22 is in fluid communication with the internal cavity 24 of the balloon 18, with the other end being in fluid communication with an internal cavity (not shown) of a remote injection port 26. The remote injection port 26 includes a silicone septum 28. At the time the adjustable gastric band 10 is implanted around a portion of the stomach, the remote injection port 26 is also implanted at a suitable location, usually within the rectus sheaths, for transcutaneous access via a Huber needle.

As is well known, the internal cavity 24, the flexible conduit 22 and the internal cavity of the remote injection port 26 are preferably at least partially filled with a physiologically compatible fluid, such as a saline solution. Postoperative adjustment of the perimeter enclosed by the balloon 18, and therefore the size of the stoma, is accomplished by addition or removal of fluid from the interior cavity 24 of the balloon 18 by inserting a Huber needle percutaneously into the silicone septum 28 of the injection port 18.

As is well known in the field the adjustable gastric band 10 may be made from any suitable medically compatible material having sufficient strength necessary for a particular laparoscopic surgery or particular patient.

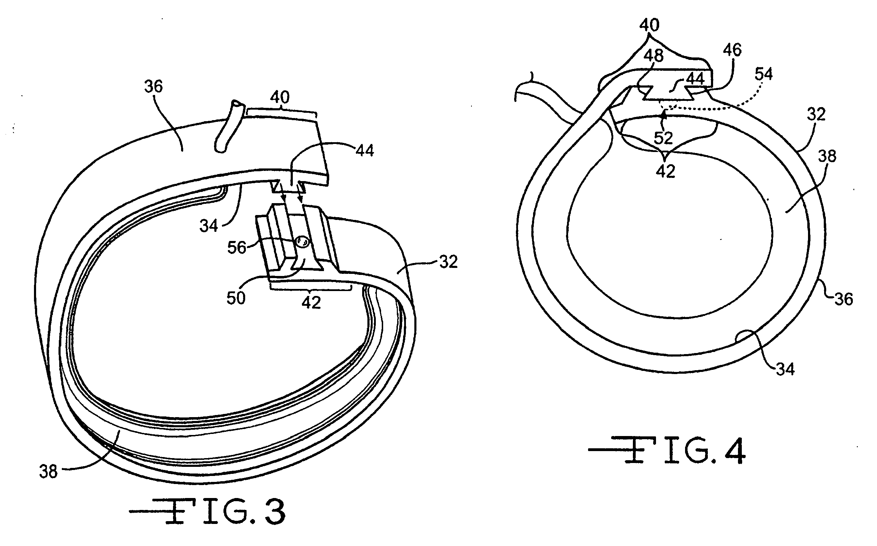

As mentioned above, the two ends of the adjustable gastric band 10 are attached together (the specific attachment mechanism structure is not illustrated in FIG. 1). The present invention is directed to various embodiments of transverse attachment mechanisms for connecting the two ends together. The general construction of adjustable gastric band 10 shown in FIGS. 1 and 2 and described above is common to the embodiments illustrated in FIGS. 3-11, with the embodiments differing by the specific attachment mechanisms. It is noted that the practice of the present invention may be used with any band, and is not limited to use with an adjustable gastric band having the exact features described above or below.

Turning now to FIGS. 3 and 4, the adjustable gastric band 30 includes an elongated strap 32 extending in what is referred to herein as the longitudinal direction, even though when implanted the adjustable gastric band 30 has an arcuate configuration. The strap 32 includes an inner surface 34 and an outer surface 36, with the balloon 38 extending inwardly from adjacent the inner surface 34. The adjustable gastric band 30 includes a first end portion 40 which overlaps a second end portion 42, with the inner surface 34 of the adjustable gastric band 30 at the first end portion 40 being disposed adjacent and outside the outer surface 36 of the adjustable gastric band 30 at the second end 42 portion.

The first and second end portions 40, 42 are secured together by a dovetail transverse attachment mechanism. A generally trapezoidally shaped member 44, which is the pin portion of the dovetail connection, oriented transverse to the longitudinal direction, extends from inner surface 34 at first end portion 40. The member 44 has three sides of a trapezoid with the opposite sides 46, 48 inclined toward each other. A complementary shaped transverse channel 50, the tail portion of the dovetail connection, is formed in the outer surface 36 at the second end portion 42, configured to receive member 44.

The transverse attachment mechanism includes a detent 52 which locates the member 44 relative to the channel 50, resists relative transverse movement therebetween, and provides positive feedback to the surgeon regarding engagement. The detent 52 is formed by a bump 54 extending from the lower end of member 44 and a complementarily shaped recess 56 formed in the base of the channel 50.

It should be appreciated that the positions of the dovetail shaped member 44 and the channel 50 could be switched, with the member 44 being formed on the outer surface 36 of second end portion 42, and the channel 50 could be formed on the inner surface 34 of first end portion 40. The positions of the bump 54 and recess 56 could also be switched.

Additionally, those skilled in the art will recognize that the trapezoidal cross section is illustrative and that many geometric shapes and quantities of member 44 and channel 50, and detent 52 may be used.

To connect the two ends together, after wrapping the adjustable gastric band 30 about the stomach, member 44 is slid into the channel 50, requiring only a nominal force to overcome detent 52. Because the relative motion between the engaged first and second end portions 40, 42 is constrained to the transverse direction until they are disengaged, any longitudinal or radial (outward) force on the adjustable gastric band 30 will not separate the end portions 40, 42 (without breakage or deformation). To separate the end portions 40, 42 only a nominal transverse force is required to produce transverse movement there between, a force greater than transverse forces which the dovetail transverse attachment mechanism would normally experience when the adjustable gastric band 30 is implanted. Once the end portions 40, 42 are disengaged they may move longitudinally.

Although the member 44 and channel 50 have been depicted as trapezoidal in shape, any shape may be used which resists the first and second end portions 40, 42 from moving longitudinally or radially once engaged, but which provide transverse movement as substantially the only direction of relative movement and force which allows the two end portions 40, 42 to be disengaged.

Since the longitudinal width of the channel 50 is narrower than the longitudinal width of a portion of the member 44, a result of the general trapezoidal shape of the member 44 and the channel 50, relative radial (outward) movement between the end portions 40, 42 is prevented. Since the transverse surfaces of the member 44 and the channel 50 are generally perpendicular to the direction of longitudinal loading (the holding force), relative transverse movement does not result from the loading. Thus, due to the orthogonal relationship between longitudinal force (holding or separating force) and only transverse force and movement being operative to disengage the ends of the adjustable gastric band decouples the longitudinal force from the removal force.

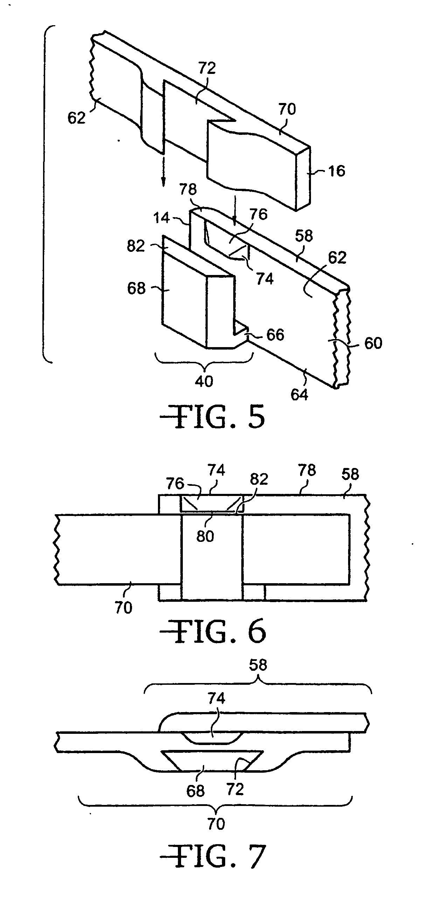

In FIG. 5, another embodiment of a dovetail transverse attachment mechanism is illustrated. A first end portion 58 of an adjustable gastric band 60 includes an U-shaped integral member 62 extending from the outer surface 64 of the adjustable gastric band 60. The U-shaped member 62 includes an outwardly extending base 66 which supports at its distal end a transversely extending member 68 which is spaced from and generally parallel to the outer surface 62, and which forms the pin portion of a dovetail connection.

As shown, member 68 has a trapezoidal shape. There is a complementarily shaped transverse channel 72 formed in the outer surface 62 of the adjustable gastric band 60 at the second end portion 70, which is configured to received the member 68.

A retaining member 74 extends from the outer surface 62 at first end portion 58, transversely aligned with the gap between the member 68 and the outer surface 62. Referring also to FIGS. 6 and 7, retaining member 74 includes inclined surface 76 beginning at the upper edge 78 of the first end portion 58 and terminating at the lower surface 80 of retaining member 74. As seen in FIGS. 6 and 7, the lower surface 80 is spaced above, but does not overlie, the distal end 82 of the member 68 leaving a gap 84 therebetween. The gap 84 is sized to allow, in conjunction with the flexibility of the adjustable gastric band 60, the second end portion 70 to be inserted therethrough, with guidance from the inclined surface 76 acting as a ramp, so that the member 68 may be disposed in the channel 72 with the second end portion 70 extending through the space between the member 68 and the outer surface 62 at the first end portion 58.

The transverse width of the second end portion 70 is less than the transverse space between the base 66 and the lower surface 80. The lower surface 80 extends generally perpendicular from the outer surface 62, and perpendicular to any relative movement between the first and second end portions 58, 70 when engaged, resisting transverse forces so as to retain the second end portion 70 in place. Because the relative movement between the first and second end portions 58, 70 is constrained to the transverse direction until they are uncoupled, longitudinal or radial forces on the adjustable gastric band 60 will not separate them.

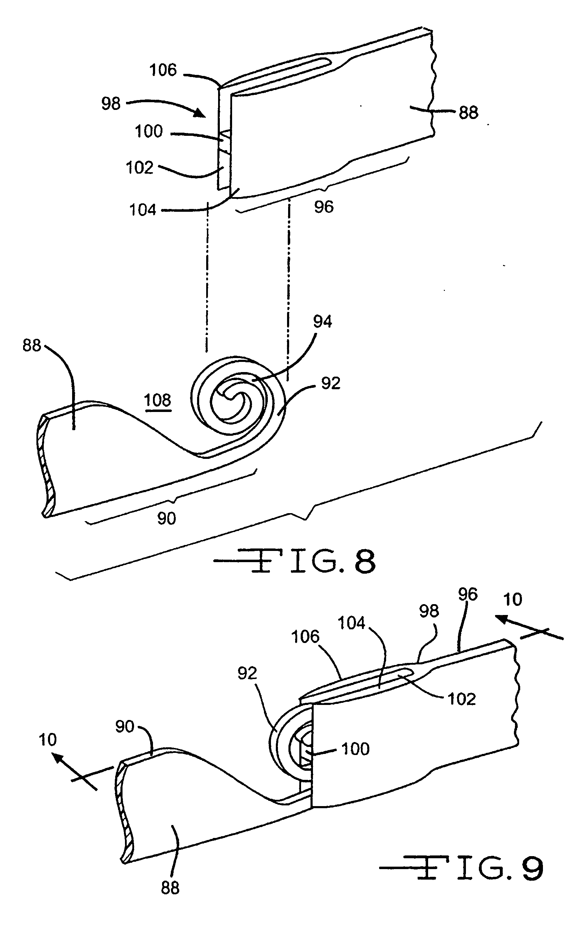

Referring to FIGS. 8 and 9, there is illustrated in perspective an adjustable gastric band with a hook transverse attachment mechanism shown unattached. The adjustable gastric band 88 includes a first end portion 90 that terminates in a spiral hook member 92 which defines a spiral gap 94 of approximately 1-2 mm. The spiral hook member 92 may be formed of metal, hard plastic, or other suitable material, and is attached to the adjustable gastric band 88 in any appropriate manner, such as with silicone adhesive, or the like. The configuration and material of hook member 92 may provide spring like qualities, which may have desirable therapeutic and performance benefits.

The second end portion 96 of adjustable gastric band 88 terminates in a buckle 98, having a catch bar 100 extending across a gap 102 defined by two spaced apart parallel flanges 104, 106.

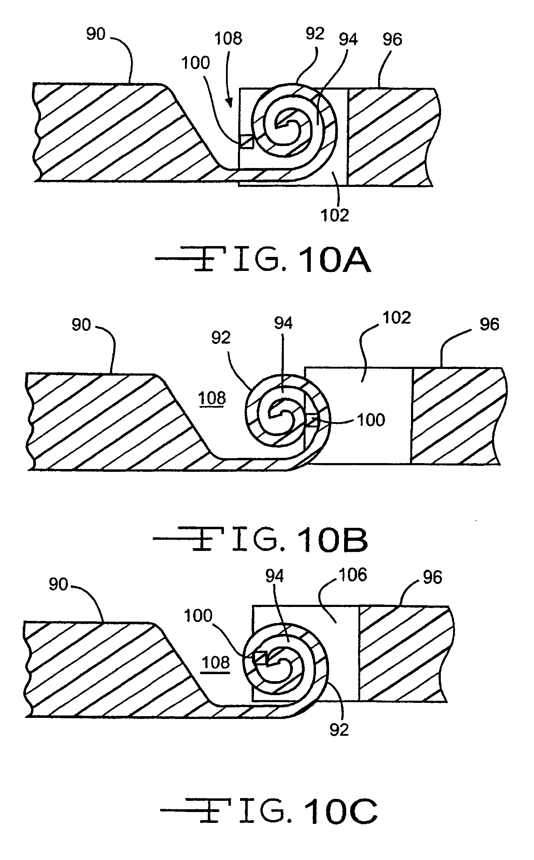

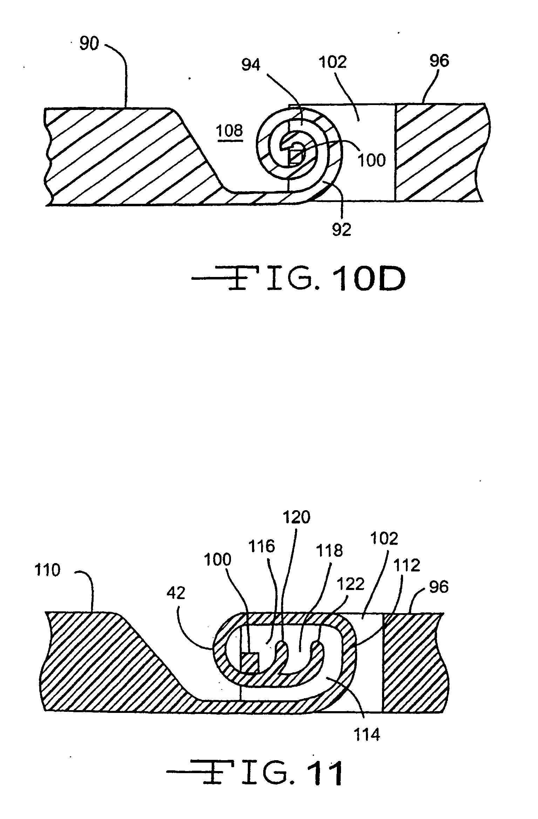

The process of engaging the first and second end portions 90, 96 is depicted in the sequence of positions illustrated in FIGS. 10A-D. The hook member 94 is initially aligned below the gap 102. In FIG. 10A, the spiral hook member 94 is disposed within the gap 102, with the catch 100 located in the entrance 108, an narrowing opening in the first end portion 90 which leads to the spiral gap 94. The catch 100 is advanced along the path of the spiral gap 94 by moving the first end portion 90 transversely and longitudinally relative to the second end portion 96, to the position shown in FIG. 10B. Further transverse and longitudinal movement of the first end portion 90 relative to the second end portion 96 places catch 100 further along the spiral gap 94, to the position shown in FIG. 10C, and ultimately in the final position shown in FIG. 10D.

In the fully engaged position of FIG. 10D, longitudinal force will not cause the engaged first and second end portions 90, 94, to separate. Disengagement requires coordinated transverse and longitudinal relative movement between the two end portions 90, 94. Although a spiral path is shown, any circuitous path may be used which requires coordinated longitudinal and transverse movement directions, to disengage the two ends portions 90, 94.

FIG. 11 illustrates an alternate embodiment of the hook member forming a longitudinally adjustable transverse attachment mechanism. The first end portion 110 terminates in the hook member 112 which defines a divergent path 114. The divergent path 114 allows selection of a desired position 116, 118 into which catch 100 may be transversely positioned. Hook member 112 terminates in spaced apart ends 120, 122 which define positions 116, 118. The configuration of hook member 112 allows the circumference of the adjustable gastric band to be set at more than one length.

Additional spaced apart ends may be incorporated to provide additional positions. The ends of the hook member may be configured to extend longitudinally so that the catch cannot be repositioned by transverse movement alone.

It will become readily apparent to those skilled in the art that the above invention has equally applicability to other types of implantable bands. For example, bands are used for the treatment of fecal incontinence. One such band is described in U.S. Patent 6,461,292 . Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729 .

Thus, as used herein and in the claims, an implantable band is a band which may be implanted in a position to occlude flow, such as food or body fluids, through an anatomical passageway, such as a stomach or lumen.

In summary, numerous benefits have been described which result from employing the concepts of the invention. The foregoing description of one or more embodiments of the invention has been presented for purposes of illustration and description. It is not intended to be exhaustive or to limit the invention to the precise form disclosed. Obvious modifications or variations are possible in light of the above teachings. The one or more embodiments were chosen and described in order to best illustrate the invention and its practical application to thereby enable one of ordinary skill in the art to best utilize the invention in various embodiments and with various modifications as are suited to the particular use contemplated. It is intended that the scope of the invention be defined by the claims appended hereto.

## Claims

1. An implantable adjustable band for treatment of a medical condition, the band comprising:
(a) a strap configured to encircle an anatomical passageway, said strap defining a circumferential direction thereabout, said strap having an inner and outer surface;
(b) first and second end portions disposed at either end of said elongated strap, said first and second end portions including respective inner and outer surfaces which correspond to said inner and outer surfaces of said strap;
(c) a transverse attachment mechanism configured to attach said first end portion to said second end portion so as to secure said strap adjacent the anatomical passageway.

2. The band of claim 1, wherein said transverse attachment mechanism comprises a transverse member formed as part of said first end portion and a transverse opening formed as part of one of said inner and outer surfaces of said second end portion, said transverse opening configured to receive said transverse member.

3. The band of claim 2, wherein said transverse attachment mechanism comprises a dovetail transverse attachment mechanism having a pin portion and a tail portion, said transverse member comprising said pin portion and said transverse opening comprising said tail portion.

4. The band of claim 3, wherein said transverse member has a generally trapezoidal shape and said transverse opening comprises a channel having a trapezoidal shape.

5. The band of claim 4, wherein said transverse attachment mechanism includes a detent, a first part of said detent being carried by said transverse member, a second part of said detent being carried by said transverse opening.

6. The band of claim 3 or claim 4, wherein said first end portion includes a U-shaped portion, said U-shaped portion comprising a base extending laterally from said first end portion and said transverse member which extends transversally from said base thereby defining a gap.

7. The band of claim 6 wherein said second end portion is configured to be disposed through said gap when said transverse member is disposed in said transverse opening.

8. The band of claim 6, comprising a retaining member extending from said first end portion overlying said gap, said retaining member configured to resist withdrawal of said second end portion from said gap.

9. The band of claim 8, wherein said retaining member includes a lower surface disposed generally perpendicular to said gap.

## Patentansprüche

1. Implantierbares verstellbares Band zur Behandlung einer medizinischen Erkrankung, worin das Band Folgendes umfasst:
(a) einen Gurt, der einen anatomischen Durchgang umkreisen kann, wobei der Gurt eine Umfangsrichtung um ihn herum definiert und eine Innen- und Außenseite aufweist;
(b) erste und zweite Endabschnitte, die an jedem Ende des länglichen Gurts angeordnet sind, wobei die ersten und zweiten Endabschnitte jeweils Innen- und Außenseiten aufweisen, die den Innen- und Außenseiten des Gurts entsprechen;
(c) einen querverlaufenden Befestigungsmechanismus, der den ersten Endabschnitt am zweiten Endabschnitt zur Fixierung des Gurts neben dem anatomischen Durchgang befestigen kann.

2. Band nach Anspruch 1, worin der querverlaufende Befestigungsmechanismus ein Querelement, das als Teil des ersten Endabschnitts geformt ist, und eine Queröffnung, die als Teil der Innen- oder der Außenseite des zweiten Endabschnitts geformt ist, umfasst, wobei die Queröffnung das Querelement aufnehmen kann.

3. Band nach Anspruch 2, worin der querverlaufende Befestigungsmechanismus einen schwalbenschwanzförmigen Befestigungsmechanismus mit einem Stiftabschnitt und einem Schwanzabschnitt umfasst, wobei das Querelement den Stiftabschnitt und die Queröffnung den Schwanzabschnitt umfasst.

4. Band nach Anspruch 3, worin das Querelement eine allgemein trapezförmige Gestalt aufweist und die Queröffnung einen Kanal mit einer trapezförmigen Gestalt umfasst.

5. Band nach Anspruch 4, worin der querverlaufende Befestigungsmechanismus eine Arretierung aufweist, wobei ein erster Teil der Arretierung vom Querelement getragen wird und ein zweiter Teil der Arretierung von der Queröffnung getragen wird.

6. Band nach Anspruch 3 oder Anspruch 4, worin der erste Endabschnitt einen U-förmigen Abschnitt aufweist, wobei der U-förmige Abschnitt einen Boden umfasst, der sich seitlich vom ersten Endabschnitt und vom Querelement erstreckt, das sich quer vom Boden erstreckt und so eine Lücke definiert.

7. Band nach Anspruch 6, worin der zweite Endabschnitt durch die Lücke angeordnet werden kann, wenn das Querelement in der Queröffnung angeordnet ist.

8. Band nach Anspruch 6, umfassend ein Halteelement, das sich vom ersten über der Lücke liegenden Endabschnitt erstreckt, wobei das Halteelement Zurückziehen des zweiten Endabschnitts aus der Lücke widerstehen kann.

9. Band nach Anspruch 8, worin das Halteelement eine allgemein senkrecht zur Lücke angeordnete Unterseite aufweist.

## Revendications

1. Bande ajustable implantable pour le traitement d'une pathologie médicale, la bande comprenant :
(a) une sangle configurée pour encercler un passage anatomique, ladite sangle définissant une direction circonférentielle autour de celle-ci, ladite sangle ayant une surface interne et une surface externe ;
(b) des première et deuxième portions d'extrémité disposées à chaque extrémité de ladite sangle allongée, lesdites première et deuxième portions d'extrémité comportant des surfaces respectives interne et externe qui correspondent auxdites surfaces interne et externe de ladite sangle ;
(c) un mécanisme de fixation transversale configuré pour fixer ladite première portion d'extrémité à ladite deuxième portion d'extrémité de manière à fixer ladite sangle en position adjacente audit passage anatomique.

2. Bande selon la revendication 1, dans laquelle ledit mécanisme de fixation transversale comprend un organe transversal formé en tant que partie de ladite première portion d'extrémité et une ouverture transversale formée en tant que partie de l'une desdites surfaces interne et externe de ladite deuxième portion d'extrémité, ladite ouverture transversale étant configurée pour recevoir ledit organe transversal.

3. Bande selon la revendication 2, dans laquelle ledit mécanisme de fixation transversale comprend un mécanisme de fixation transversale en queue d'aronde ayant une portion de goupille et une portion de queue, ledit organe transversal comprenant ladite portion de goupille et ladite ouverture transversale comprenant ladite portion de queue.

4. Bande selon la revendication 3, dans laquelle ledit organe transversal a une forme généralement trapézoïdale et ladite ouverture transversale comprend un canal ayant une forme trapézoïdale.

5. Bande selon la revendication 4, dans laquelle ledit mécanisme de fixation transversale comporte un ergot, une première partie dudit ergot étant portée par ledit organe transversal, une deuxième partie dudit ergot étant portée par ladite ouverture transversale.

6. Bande selon la revendication 3 ou 4, dans laquelle ladite première portion d'extrémité comporte une portion en forme de U, ladite portion en forme de U comprenant une base s'étendant latéralement depuis ladite première portion d'extrémité et ledit organe transversal qui s'étend transversalement depuis ladite base en définissant ainsi un espace.

7. Bande selon la revendication 6, dans laquelle ladite deuxième portion d'extrémité est configurée de manière à être disposée à travers ledit espace lorsque ledit organe transversal est disposé dans ladite ouverture transversale.

8. Bande selon la revendication 6, comprenant un organe de retenue s'étendant depuis ladite première portion d'extrémité recouvrant ledit espace, ledit organe de retenue étant configuré pour résister au retrait de ladite deuxième portion d'extrémité dudit espace.

9. Bande selon la revendication 8, dans laquelle ledit organe de retenue comporte une surface inférieure disposée généralement perpendiculairement audit espace.
